(19) Europäisches Patentamt
European Patent Office
Office européen des brevets

(11) Veröffentlichungsnummer: **0 207 421 B1**

(12) # EUROPÄISCHE PATENTSCHRIFT

(45) Veröffentlichungstag der Patentschrift: **30.09.92**

(51) Int. Cl.$^5$: **A61K 31/015**

(21) Anmeldenummer: **86108549.6**

(22) Anmeldetag: **23.06.86**

(54) **Verwendung eines Retinoids zur Herstellung eines antikeratinisierenden Arzneimittels.**

(30) Priorität: **28.06.85 CH 2753/85**

(43) Veröffentlichungstag der Anmeldung:
**07.01.87 Patentblatt 87/02**

(45) Bekanntmachung des Hinweises auf die Patenterteilung:
**30.09.92 Patentblatt 92/40**

(84) Benannte Vertragsstaaten:
**BE CH DE FR GB IT LI LU NL SE**

(56) Entgegenhaltungen:

**FR-A- 2 529 458**

**ROUX'S ARCHIVES OF DEVELOPMENTAL GIOLOGY, Band 194, 1984, Springer Verlag; A.KISTLER, Seiten 9-17***

**M.B. SPORN et al., "The Retinoids", Band 1, 1984, Academic Press, Inc.; Seiten 235-279, 408***

**FORTSCHR. MED., Band 103, Nr. 26, 1985; W.BOLL, Seiten, 691/31-693/38***

**ARCHIVES OF DERMATOLOGICAL RESEARCH, Band 257-258, Springer Verlag; F.OTT et al., Seiten 257-258***

**THE JOURNAL OF PHARMACOLOGY AND EXPERIMENTAL THERAPEUTICS, Band 237, Nr. 1, The American Society for Pharmacology and Experimental Therapeutics (US); M.J.CONNOR et al., Seiten 31-35***

**R. BERKOW, "The Merck Manual of Diagnosis and Therapy", 14. Auflage, 1982, Merck Sharp & Dohme Research Laboratories, Rahway, NJ (US); Seiten 2017-2019***

(73) Patentinhaber: **F. HOFFMANN-LA ROCHE AG**
**Postfach 3255**
**CH-4002 Basel(CH)**

(72) Erfinder: **Bollag, Werner, Dr.**
**Mythenstrasse 10**
**CH-4054 Basel(CH)**

(74) Vertreter: **Lederer, Franz, Dr. et al**
**Lederer, Keller & Riederer Patentanwälte**
**Lucile-Grahn-Strasse 22**
**W-8000 München 80(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Im Rahmen der vorliegenden Erfindung wurde festgestellt, dass das 1, 2, 3, 4-Tetrahydro-1, 1, 4, 4-tetramethyl - 6 (α-methylstrylyl)naphtalin der Formel

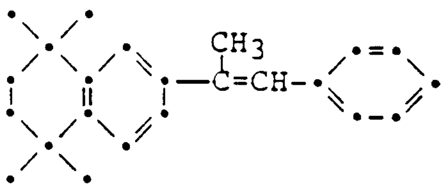

I

antikeratinisierende Eigenschaften besitzt. Es ist bekannt, die Verbindung der Formel I zur systemischen und topischen Behandlung bzw. Verhütung von Erscheinungen zu verwenden, die auf einer erhöhten Talg-Ausscheidung beruhen, wie fettiges Haar, fettige Kopfhaut, Soborrhoe und insbesondere Akne vulgaris, vgl. Roux's Arch. Dev. Biol. (1984) 194: 9 - 17 und FR-A-2 529 458.

In Sporn et al., The Retinoids, Bd. 1, 1984, S. 266 und J. Pharmacol. Exp. Ther. 237, Nr. 1, S. 32 wird die Verbindung I ohne herausragende pharmakologische Eigenschaften erwähnt.

Ueberraschend wurde nun gefunden, dass durch systemische und topische Verabreichung der Verbindung der Formel I andere pathologische Zustände als die zuletzt erwähnten erfolgreich behandelt bzw. verhütet werden können. Die vorliegende Erfindung basiert auf dem eben genannten Befund und betrifft demnach die neue Verwendung der Verbindung der Formel I zur Herstellung von Arzneimitteln für die Behandlung bzw. Verhütung von mit einer pathologischen Verhornung einhergehenden Dermatosen Beispiele solcher Dermatosen sind alle Formen von Psoriasis, z.B. Psoriasis vulgaris, Psoriasis pustulosa, Psoriasis erythrodermica, Psoriasis arthropathica, Parapsoriasis, Palmoplantare Pustulose, alle Formen von Ichthyosen, z.B. Ichthyosis vulgaris und kongenitale Ichthyosen, Keratodermien aller Art, z.B. palmoplantare Keratodermie, andere Genodermatosen mit pathologischen Verhornungsstörungen, z.B. Morbus Darier, ferner Lichen ruber planus und Pityriasis rubra pilaris.

Fur die erfindungsgemässe Verwendung ist das (E)-Isomere, d.h. das 1,2,3,4-Tetrahydro-1,1,4,4-tetramethyl -6-[(E)-α-methylstyryl)naphthalin (Verbindung Ia), bevorzugt, obwohl verauszusehen ist, dass das (Z)-Isomere, d.h. das 1,2,3,4-Tetrahydro-1,1,4,4-tetramethyl -6-[(Z)-α-methylstyryl]naphthalin (Verbindung Ib) im Rahmen der vorliegenden Erfindung ebenfalls verwendet werden kann, sei es allein oder in Kombination mit dem bevorzugten (E)-Isomeren Ia.

Zur Behandlung bzw. Verhütung der oben genannten Krankheiten wird die Verbindung der Formel I systemisch oder topisch, vorzugsweise systemisch, besonders bevorzugt enteral, ganz besonders bevorzugt oral, verabreicht.

Die Dosis bei der systemischen Verabreichung variiert gemäss den Bedürfnissen des einzelnen Patienten, wie sie vom behandelnden Arzt bestimmt worden sind. Im allgemeinen dürfte jedoch eine tägliche Dosis von 1 mg bis 50 mg, vorzugsweise von 3 mg bis 15 mg, pro kg Körpergewicht des Patienten zum Einsatz gelangen. Die Dosis kann als Einzeldosis oder auf mehrere Teildosen verteilt gemäss einem Dosierungsplan, wie er vom Arzt gemäss den Bedürnissen des Patienten bestimmt wird, verabreicht werden.

Als Verabreichungsform kommen die für die systemische Verabreichung üblichen festen oder flüssigen Darreichungsformen in Betracht, z.B. Suppositorien oder als feste orale Darreichungsformen Kapseln, Tabletten, Dragées, Pillen, Puder, Granulate und dergleichen, als flüssige orale Darreichungsformen Lösungen, Sirupe, Suspensionen, Elixire und dergleichen und als parenterale Darreichungsformen Infusions- oder Injektionslösungen, die intravenös oder intramuskulär injiziert werden können.

Es liegt im Bereich der vorliegenden Erfindung, die Verbindung der Formel I in jeder für die systemische Verabreichung geeigneten Menge in die enterale oder parenterale Darreichungsform einzuarbeiten. Es ist jedoch bevorzugt, Präparate herzustellen, die den erfindungsgemässen Wirkstoff in einer Menge von 50-1000 mg, vorzugsweise von 150-500 mg, enthalten. Besonders bevorzugt ist die Herstellung von Kapseln und Tabletten.

Zur topischen Verabreichung geeignet sind Lösungen, Lotions, Suspensionen, Salben, Crèmes, Gels, mikronisierte Puder, Aerosole und dergleichen. Diese Präparate enthalten zweckmässig 0,1-10 Gew.%, vorzugsweise 0,5-5 Gew.% der Verbindung der Formel I berechnet auf das Gesamtgewicht des Präparates.

Die Herstellung der oben genannten systemischen und topischen Gebrauchsformen kann in üblicher Weise, z.B. anhand der nachstehenden Beispiele erfolgen.

Beispiel 1

Hartgelatinekapseln enthaltend die folgenden Bestandteile können hergestellt werden:

| Bestandteile | mg/Kapsel |
|---|---|
| 1. sprühgetrocknetes Pulver enthaltend 75% Verbindung Ia | 200 |
| 2. Natriumdioctylsulfosuccinat | 0,2 |
| 3. Natriumcarboxymethylcellulose | 4,8 |
| 4. mikrokristalline Cellulose | 86,0 |
| 5. Talk | 8,0 |
| 6. Magnesiumstearat | 1,0 |
| | Total 300 |

Verfahren:

Das sprühgetrocknete Pulver, das auf dem Wirkstoff, Gelatine und mikrokristalliner Cellulose basiert und eine mittlere Korngrösse des Wirkstoffes von <1 $\mu$m aufweist (mittels Autokorrelationsspektroskopie gemessen), wird mit einer wässrigen Lösung von Natriumcarboxymethylcellulose und Natriumdioctylsulfosuccinat befeuchtet und geknetet. Die resultierende Masse wird granuliert, getrocknet und gesiebt, und das erhaltene Granulat mit mikrokristalliner Cellulose, Talk und Magnesiumstearat vermischt. Das Pulver wird in Kapseln der Grösse 0 abgefüllt.

Beispiel 2

Tabletten, enthaltend die folgenden Bestandteile, können hergestellt werden:

| Bestandteile | mg/Tablette |
|---|---|
| 1. Verbindung Ia als feingemahlenes Pulver | 500 |
| 2. Milchzucker pulv. | 100 |
| 3. Maisstärke weiss | 60 |
| 4. Povidone® K30 | 8 |
| 5. Maisstärke weiss | 112 |
| 6. Talk | 16 |
| 7. Magnesiumstearat | 4 |
| | Total 800 |

Verfahren:

Die feingemahlene Substanz wird mit Milchzucker pulv. und Maisstärke weiss gemischt. Die Mischung wird mit einer wässrigen Lösung von Providone® K30 befeuchtet und geknetet, und die resultierende Masse granuliert, getrocknet und gesiebt. Das Granulat wird mit Maisstärke weiss (2. Teil), Talk und Magnesiumstearat vermischt und zu Tabletten geeigneter Grösse verpresst.

Beispiel 3

Weichgelatinekapseln, enthaltend die folgenden Bestandteile, können hergestellt werden:

| Bestandteile | mg/Kapsel |
|---|---|
| 1. Verbindung Ia | 50 |
| 2. Triglycerid | 450 |
| | Total 500 |

Verfahren:

10 g Verbindung Ia werden unter Rühren, Inertbegasung und Lichtschutz in 90 g mittelkettigem Triglycerid gelöst. Diese Lösung wird als Kapselfüllmasse von einem Vertragshersteller zu Weichgelatinekapseln à 50 mg Wirkstoff verarbeitet.

Beispiel 4

Eine Fettsalbe, enthaltend die folgenden Bestandteile, kann hergestellt werden:

| Bestandteile | |
|---|---|
| 1. Verbindung Ia, feingemahlen | 3,0 g |
| 2. Paraffinöl, dickflüssig | 30,0 g |
| 3. Lunacera® M | 15,0 g |
| 4. Ricinusöl, gehärtet | 5,0 g |
| 5. Vaseline, weiss | ad 100 g |

Verfahren:

Sämtliche Hilfsstoffe werden in der Wärme gemischt und auf Raumtemperatur kaltgerührt. Der Wirkstoff wird kalt unter Lichtschutz mit der auf diese Weise erhaltenen Mischung homogen angerieben.

Beispiel 5

Eine Fettcrème, enthaltend die folgenden Bestandteile, kann hergestellt werden:

| Bestandteile | | |
|---|---|---|
| 1. Verbindung Ia, feingemahlen | 3,0 g | |
| 2. Vaseline, weiss | 30,0 g | Fettphase |
| 3. Wachs, weiss | 5,0 g | Fettphase |
| 4. Paraffinöl, dickflüssig | 20,0 g | Fettphase |
| 5. Dehymuls® E | 9,0 g | Wasserphase |
| 6. Benzoesäure | 0,2 g | Wasserphase |
| 7. entmineralisiertes Wasser | ad 100,0 g | Wasserphase |

Verfahren:

Fett- und Wasserphase werden zu einer Fettcrème verarbeitet. Der Wirkstoff wird bei Raumtemperatur unter Lichtschutz mit dieser Fettcrème homogen angerieben.

Beispiel 6

Eine Vanishingcrème (Typ O/W-Emulsion), enthaltend die folgenden Bestandteile, kann hergestellt werden:

| Bestandteile | | | |
|---|---|---|---|
| 1. Verbindung Ia, feingemahlen | | 3,0 g | |
| 2. Glycerinmonostearat | | 17,0 g | Fettphase |
| 3. Deltyl® extra | | 4,0 g | Fettphase |
| 4. Tween® 60 | | 4,0 g | Fettphase |
| 5. Span® 60 | | 4,0 g | Fettphase |
| 6. Silikonöl AR 20 | | 2,0 g | Fettphase |
| 7. Propylenglykol | | 10,0 g | Wasserphase |
| 8. Benzoesäure, rein | | 0,2 g | Wasserphase |
| 9. entmineralisiertes Wasser | ad | 100,0 g | Wasserphase |

Verfahren:

Fett- und Wasserphase werden zu einer Crème verarbeitet. Der Wirkstoff wird bei Raumtemperatur unter Lichtschutz mit dieser Crème homogen angerieben.

Beispiel 7

Ein hydrophiles Gel, enthaltend die folgenden Bestandteile, kann hergestellt werden:

| Bestandteile | |
|---|---|
| 1. Verbindung Ia, feingemahlen | 3,0 g |
| 2. Carbopol® 940 | 2,5 g |
| 3. Propylenglykol | 50,0 g |
| 4. Aethanol, 94% | ad 100 g |

Verfahren:

Der Wirkstoff wird unter Lichtschutz in die Mischung Propylenglykol/Aethanol, 94%ig, eingearbeitet. Carbopol® 940 wird bis zu vollständigen Gelierung eingerührt.

Beispiel 8

Eine Lotion, enthaltend die folgenden Bestandteile, kann hergestellt werden:

| Bestandteile | |
|---|---|
| 1. Verbindung Ia, feingemahlen | 3,0 g |
| 2. Carbopol® 934 | 0,6 g |
| 3. Natriumhydroxid | q.s. ad pH 6 |
| 4. Aethanol, 94% | 50,0 g |
| 5. entmineralisiertes Wasser | ad 100,0 g |

Verfahren:

Der Wirkstoff wird unter Lichtschutz in die Mischung Aethanol, 94%ig/Wasser eingearbeitet. Carbopol® 934 wird bis zur vollständigen Gelierung eingerührt und der pH-Wert mit Natriumhydroxid eingestellt.

Die therapeutische und prophylaktische Wirkung der Verbindung der Formel I bei mit einer pathologischen Verhornung einhergehenden Dermatosen kann den nachfolgenden Versuchen entnommen werden:

A) Die antikeratinisierende Wirkung der Verbindung Ia wurde nach dem folgenden Prozedere am Rhinomausmodell bestimmt. Die Haut der Rhinomaus zeichnet sich durch das Vorhandensein von mit Keratin angefüllten Utriculi der Epidermis und subkutanen Zysten aus, welche beide von Haarfollikeln herrühren. Die Verabreichung von Retinoiden führt zu einer Hyperproliferation der Epidermis und der epithelialen Auskleidung der Utriculi. Die Verdickung der Epidermis und die Reduktion der Grösse der Utriculi führen zu einer Normalisierung der veränderten Struktur der Epithelschicht. Die tägliche topische Applikation von 0,1 ml/$cm^2$ Haut der Rhinomaus von einer 3%igen Acetonlösung der Verbindung Ia während 3 Wochen bzw. die dreimalige wöchentliche orale Verabreichung von Verbindung Ia in Arachisöl während 3 Wochen führt zu einer signifikanten Proliferation der Epidermis und markanten Verkleinerung der mit Keratin angefüllten Utriculi. Die Resultate sind in den Tabellen I und II zusammengefasst.

## Tabelle I

Topische Anwendung der Verbindung Ia während 3 Wochen

| Behandlung | Mit Keratin gefüllte Utriculi | | |
|---|---|---|---|
| Tägliche topische Anwendung von 0,1 ml/$cm^2$ | Ø D1 des Utriculus in µm (gemessen an der weitesten Stelle) | Ø D2 des Utriculus in µm (gemessen an der Oeffnung) | D1/D2 |
| Aceton | 154,0 | 74,4 | 2,2 |
| Verbindung Ia 3%ig in Aceton | 76,9 | 59,7 | 1,3 |

Tabelle II

Systemische Anwendung der Verbindung Ia während 3 Wochen

| Behandlung | Mit Keratin gefüllte Utriculi | | |
|---|---|---|---|
| 3x wöchentlich oral | Ø D1 des Utriculus in µm (gemessen an der weitesten Stelle) | Ø D2 des Utriculus in µm (gemessen am der Oeffnung) | D1/D2 |
| Arachisöl | 135,1 | 71,5 | 1,9 |
| Verbindung Ia | | | |
| 25 mg/kg | 116,8 | 72,4 | 1,6 |
| 75 mg/kg | 90,2 | 62,2 | 1,4 |
| 225 mg/kg | 88,7 | 67,2 | 1,3 |

Es ist bekannt, dass das 1,2,3,4-Tetrahydro-1,1,4,4--tetramethyl -6-[(E)-α-methylstyryl]naphthalin Ia gut vertragen wird, und dass in den therapeutisch angezeigten Dosen keine toxischen Symptome auftreten. So zeigt die Verbindung Ia insbesondere keine Erscheinungen der Hypervitaminose A (z.B. keine Manifestationen an Haut und Schleimhäuten), und ist nicht teratogen und nicht hautirritierend. Darüber hinaus hat sich gezeigt, dass die Verbindung Ia, als einziges therapeutisch wirksames Retinoid, keine Erhöhung der Lipidwerte im Blutplasma verursacht, sondern vielmehr sogar eine lipidsenkende Wirkung besitzt.

Zum Nachweis dieser Wirkung wurden pro Dosis je 5 männlichen und weiblichen Albinoratten (Füllinsdorf/Schweiz) von ca. 150 g Gewicht mittels Schlundsonde innerhalb von 2 Tagen in Abständen von jeweils 18 und 6 Stunden fünfmal eine Dosis von Verbindung Ia verabreicht. 18 Stunden nach der letzten Applikation wurde den Versuchstieren retroorbital 1-1,2 ml Blutplasma entnommen. Der Triglyceridwert wurde durch enzymatische Spaltung der Triglyceride mit nachfolgender Bestimmung des entstandenen Glycerins (Farbreaktion) mittels Peridochrom Triglyceride GPO-PAP bestimmt. Die Resultate sind in der Tabelle V zusammengefasst.

Tabelle V

| | männliche Ratten | weibliche Ratten |
|---|---|---|
| Kontrolltiere | 201±39 mg/100 ml | 69±28 mg/100 ml |
| Verbindung der Formel Ia | | |
| 10 mg/kg | 158±56 mg/100 ml | 56±25 mg/100 ml |
| 100 mg/kg | 173±18 mg/100 ml | 72±18 mg/100 ml |
| 1000 mg/kg | 41±19 mg/100 ml | 30± 8 mg/100 ml |

Die klinische Wirksamkeit von Verbindung Ia wird mit den folgenden Beispielen illustriert:

7 Patienten mit Psoriasis vulgaris und 1 Patient mit Psoriasis pustulosa wurden während 12-16 Wochen mit einer täglichen oralen Dosis zwischen 96 und 300 mg Verbindung Ia behandelt. Die zu Beginn der Behandlung bestehenden psoriatischen Läsionen (Erythem, Infiltration, Schuppung, Pusteln) verminderten sich im Verlauf der Behandlung an Intensität und Ausdehnung um 30-60%. Ausser den objektiv feststellbaren Remissionen wurden auch die subjektiven Symptome der Patienten deutlich gebessert. Im Gegensatz zu allen bisher klinisch angewandten Retinoiden verursachte Verbindung Ia keinerlei Symptome einer Hypervitaminose A und auch keine anderen Nebenerscheinungen. So wurden insbesondere keinerlei objektive oder subjektive Symptome an Haut, Haaren, Schleimhäuten, Knochen, Gelenken, Muskeln oder Nervensystem, festgestellt. Sämtliche Laborwerte (Hämatologie, Blutchemie, Urinanalyse) blieben im Verlauf der Behandlung im Bereich der Ausgangswerte. Insbesondere zeigten die Bestimmungen der Transaminasen, der alkalischen Phophatase, des Cholesterins und der Triglyzeride keine abnormen Veränderungen.

1 Patient mit chronischem Ekzem an allen Körperstellen, der seit 3 Jahren alle 2-4 Wochen einen massiven Schub der Krankheit bekam, zeigte unter der täglichen Behandlung mit 192 mg Verbindung Ia oral während 3 Monaten eine sehr gute Wirkung, wobei überhaupt keine Schübe mehr auftraten. Der Patient zeigte keinerlei Nebenerscheinungen.

**Patentansprüche**

1. Verwendung von 1,2,3,4-Tetrahydro-1,1,4,4-tetramethyl -6-(α-methylstyryl)naphthalin zur Herstellung eines Mittels zur Behandlung bzw. Verhütung von mit einer pathologischen Verhornung einhergehenden Dermatosen.

2. Verwendung gemäss Anspruch 1, dadurch gekennzeichnet, dass es sich um Psoriasis handelt.

3. Verwendung von 1,2,3,4-Tetrahydro-1,1,4,4-tetramethyl -6-[(E)-α-methylstyryl]naphthalin nach einem der Ansprüche 1 oder 2.

**Claims**

1. The use of 1,2,3,4-tetrahydro-1,1,4,4-tetramethyl-6-(α-methylstyryl)naphthalene for the manufacture of a medicament for the treatment or prevention of dermatoses which are accompanied by a pathological cornification.

2. The use in accordance with claim 1, characterized in that the dermatosis is psoriasis.

3. The use of 1,2,3,4-tetrahydro-1,1,4,4-tetramethyl-6-[(E)-α-methylstyryl]naphthalene according to either of claims 1 and 2.

## Revendications

1. Utilisation du 1,2,3,4-tétrahydro-1,1,4,4-tétraméthyl-6-(α-méthylstyryl)-naphtalène pour la préparation d'un médicament pour le traitement ou la prévention des dermatoses s'accompagnant d'une kératinisation pathologique.

2. Utilisation selon la revendication 1, caractérisée en ce qu'il s'agit du psoriasis.

3. Utilisation du 1,2,3,4-tétrahydro-1,1,4,4-tétramethyl-6-[(E)-α-méthylstyryl]-naphtalène selon l'une des revendications 1 ou 2.